# EUROPÄISCHE PATENTSCHRIFT

(11) **EP 1 209 470 B1**
(45) Veröffentlichungstag und Bekanntmachung des Hinweises auf die Patenterteilung: **21.05.2008**
(21) Anmeldenummer: 01123418.4
(22) Anmeldetag: 28.09.2001
(51) Int. Cl.: G01N 33/68, G01N 33/543

(54) **Verfahren zum Nachweis biologischer Moleküle**
Process for the detection of biological molecules
Procédé de détection des molécules biologiques

(30) Priorität: 12.10.2000 DE 10050632
(43) Veröffentlichungstag der Anmeldung: 29.05.2002
(73) Patentinhaber: Biosensor GmbH, 53175 Bonn (DE)
(72) Erfinder: Schmid, Beate, Dr., 53111 Bonn (DE); Hoffmann, Daniel, Dr., 53229 Bonn (DE); Wefing, Stefan, Dr., 53113 Bonn (DE); Schnaible, Volker, Dr., 53177 Bonn (DE); Quandt, Eckhard, Dr., 53177 Bonn (DE); Tewes, Michael, Dr., 50354 Hürth (DE); Famulok, Michael, Prof.Dr., 53175 Bonn (DE)
(74) Vertreter: Haber, Jan Wilhelm

(56) Entgegenhaltungen:
- WO-A-98/54751
- DE-C- 19 618 812
- US-A- 5 283 037
- US-A- 5 325 704
- US-A- 5 770 857
- "Love wave acoustic immunosensor operating in liquid." SENSORS AND ACTUATORS A (PHYSICAL) (JUNE 1997) VOL.A61, NO.1-3, P.279-86. 21 REFS. DOC. NO.: S0924-4247(97)01352-6 PUBLISHED BY: ELSEVIER, XP004092233
- BENDER F ET AL: "LOVE-WAVE BIOSENSORS USING CROSS-LINKED POLYMER WAVEGUIDES ON LITAO3 SUBSTRATES" ELECTRONICS LETTERS, IEE STEVENAGE, GB, Bd. 36, Nr. 19, 14. September 2000 (2000-09-14), Seiten 1672-1673, XP001143131 ISSN: 0013-5194

## Beschreibung

Die vorliegende Erfindung betrifft ein Verfahren zum Nachweis eines in einer biologischen Probe befindlichen und in molekularer Form vorliegenden Analyten, wobei ein Sensor mit einem den Analyten bindenden Substrat versehen und in einem ersten Verfahrensschritt der Sensor mit dem Analyten beaufschlagt wird, und wobei zum quantitativen Nachweis in einem nachfolgenden Verfahrensschritt die vom Sensor gebundene Gesamtmasse des Analyten gemessen wird.

Im Zuge der Entschlüsselung des humanen Genoms und anderer Genome, wurden Techniken entwickelt, um die mRNA-Expression von Zellen unter verschiedenen Bedingungen - wie zum Beispiel krank und gesund - schnell und einfach zu messen. Allerdings gibt die mRNA-Expression kein vollständiges Bild vom Zellgeschehen auf der entscheidenden Ebene der Proteine: Die Protein-Expression wird nicht nur über die Expression der mRNA geregelt, sondern zusätzlich über andere Mechanismen, zudem zeigen Proteine häufig post-translationale Modifikationen (PTMs), die spezifisch sind für einen Gewebetyp oder einen Zellzustand, und die sich auf der Ebene der mRNA nicht untersuchen lassen. Das Proteom einer Zelle oder eines Gewebes, das heisst die Arten und Mengen aller darin vorkommenden Proteine, ist daher informationsreicher als das Muster der mRNA-Expression und erlaubt eine genauere Beschreibung des Zustandes der Zelle oder des Gewebes. Deshalb sind Proteomanalysen von grossem Nutzen in der medizinischen Diagnostik, beim Auffinden von Zielproteinen für therapeutische Wirkstoffe und bei der Entwicklung der Wirkstoffe selbst.

Die einzige heute gebräuchliche Methode zur Analyse von Proteomen ist ein ZweiSchritt-Verfahren. Dabei wird das Proteomen zuerst aufgetrennt in isoelektrischem Punkt und Größe der vorhandenen Proteine mit Hilfe der zweidimensionalen Gel-Elektrophorese (2DGE). Danach werden die Proteine massenspektrometrisch (MS) charakterisiert. Diese Methode hat mehrere Nachteile. Die größten Nachteile sind, daß die reproduzierbare Herstellung hochauflösender Gele aufwändig ist, daß gewisse Proteine (zum Beispiel sehr basische oder sehr grosse Proteine) meist ausgeblendet werden, daß schwach exprimierte Proteine nicht detektiert werden, daß die eindeutige Zuordnung von Spots mit einzelnen Proteinen nicht immer möglich ist und daß bei der Auftrennung auf dem Gel Proteine gewöhnlich entfaltet werden, was nachfolgende Untersuchungen der 3D-Struktur und Funktion erschwert.

Harding et al. (1997) offenbaren einen Biosensor in Form eines Love-Wave-Sensors, dessen akustische Oberfläche mit einem Biomolekül beschichtet ist, welches spezifisch an einen Analyten bindet. Wenn der Biosensor mit einer den Analyten enthaltenden Lösung in Verbindung gebracht wird, bewirkt die Bindung des Analyten durch das spezifische Biomolekül eine Änderung der Schallwellengeschwindigkeit der akustischen Oberflächenwellen, die als Frequenzänderung detektiert werden kann. Auf diese Weise kann die Konzentration des Analyten in der Lösung bestimmt werden.

Aus der DE 196 18 812 C1 und der US 5,283,037 sind ebenfalls Sensoren zum quantitativen Nachweis von Biomolekülen bekannt, die mit immunochemisch aktiven Molekülen beschichtet sind, um über die Bindung eines Analyten an diese Moleküle die Konzentration des Analyten in einer Lösung zu bestimmen.

Die US 5,325,704 offenbart eine Sensoranordnung zur gleichzeitigen Detektion von unterschiedlichen chemischen Substanzen in Dämpfen, welche eine Vielzahl von Oberflächenschallwellensensoren umfasst.

Die US 5,770,857 offenbart ein Massenspektrometer zur Bestimmung der Masse von mehrfach geladenen Molekülen mit hohem Molekulargewicht.

Aus der WO 98/54751 A1 ist ein Verfahren zur Vorbereitung von Proben für die Verdampfung und massenspektrometrische Analyse von nichtflüchtigen Molekülen mit hohem Molekulrgewicht bekannt.

Aufgabe der der Erfindung ist es, ein Verfahren der eingangs genannten Art zu schaffen, mit dem biologische Moleküle einfach und komfortabel qualitativ analysiert werden können.

Diese Aufgabe wird mit einem Verfahren nach dem Oberbegriff des Anspruch 1 gelöst, das sich dadurch auszeichnet, daß zum qualitativen Nachweis in einem weiteren Schritt das molekulare Gewicht des die Gesamtmasse bildenden Analyten direkt auf dem Sensor mittels eines Massenspektrometers gemessen wird.

Der Gegenstand der Erfindung ist somit ein Verfahren zur schnellen Detektion und Identifikation willkürlich ausgewählter Moleküle in biologischen Proben und zur Bestimmung der Mengen jener Moleküle in diesen Proben. Dabei können einzelne Molekülsorten detektiert werden, aber auch ganze Molekül-Klassen, zum Beispiel Proteine, die eine bestimmte Domäne gemeinsam haben. Das Verfahren kann auch genutzt werden, um Wechselwirkungen zwischen Proteinen oder zwischen Proteinen und anderen Molekülen aufzuklären. Die Methode eignet sich zur schnellen und zuverlässigen Proteomanalyse und ist insbesondere für medizinische Praxis und pharmazeutische Industrie von hohem wirtschaftlichem Wert.

Das erfindungsgemässe Verfahren erlaubt eine gezielte und schnelle Analyse von spezifischen, durch den Benutzer gewählten Ausschnitten des Proteoms. Dabei werden die Nachteile der herkömmlichen Verfahren vermieden. Da das erfindungsgemässe Verfahren unter nativen Bedingungen funktioniert, bleiben Proteine strukturell und funktionell intakt. Ausserdem gibt es bei der Detektion keine Massengrenze nach oben. Daher erlaubt das erfindungsgemässe Verfahren auch die Untersuchung von Protein-Komplexen und Ähnlichem und ermöglicht damit die Charakterisierung von Protein-Wechselwirkungen und sonstigen Wechselwirkungs-Netzen. Das erfindungsgemässe Verfahren eröffnet damit neue Anwendungen.

Das erfindungsgemässe Verfahren besteht aus den folgenden Schritten oder aus einer Teilfolge dieser Schritte:

### 1. Auswahl der Liganden:

Ausgehend von einer biomedizinischen Frage werden geeignete Proteine, Protein-Domänen, Epitope, Metabolite oder ähnliches ("Liganden") ausgewählt, deren Anteile am Proteom oder Zellinhalt bestimmt werden soll. Auswahlkriterien können zum Beispiel sein die Schlüsselstellung in metabolischen oder regulatorischen Netzen oder die starke mRNA-Expression. Die Auswahl erfolgt manuell oder durch bioinformatische Analyse von biologischen Netzwerken oder sonstigen Daten.

### 2. Herstellung der Liganden:

Die ausgewählten Liganden werden nach an sich bekannten Verfahren hergestellt. So werden Proteine exprimiert und gereinigt. Falls in Schritt 1 sonstige Liganden (z.B. Metabolite) ausgewählt wurden, werden diese Moleküle zum Beispiel durch chemische Synthese oder durch Extraktion aus biologischem Material bereitgestellt.

### 3. Herstellung der Rezeptoren:

Es werden "Rezeptoren" hergestellt, die fest an die ausgewählten Liganden binden. Rezeptoren können zum Beispiel Aptamere aus Oligonukleotiden oder Antikörper sein. Die Herstellung solcher Rezeptoren ist an sich bekannt (siehe zum Beispiel Tuerk & Gold 1990 Science 249:505, Ellington & Szostak 1990 Nature 346:818).

### 4. Herstellung der Anordnung von Sensoren:

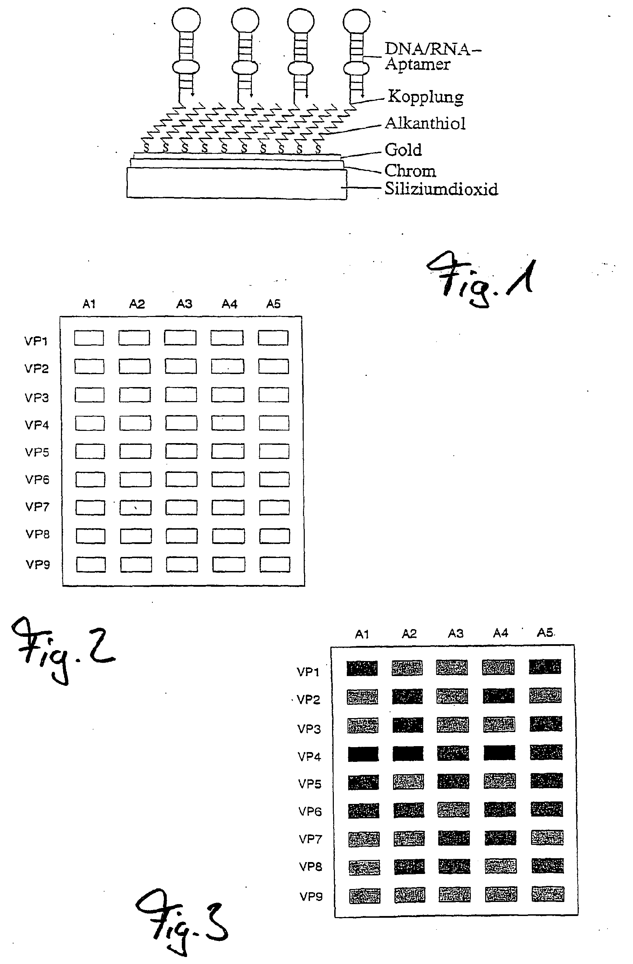

Als Sensoren werden Mikrowaagen zum Beispiel sogenannte Love-Wave-Sensoren (Harding et al 1997 Sensors and Actuators A 61:279; Abb. 1) verwendet. Mehrere Sensoren können als Array auf einer gemeinsamen Unterlage angeordnet werden. Im Falle von Love-Wave-Sensoren wird die Masse eines aufgebrachten Analyten gemessen durch Änderungen im Frequenzspektrum von Oberflächen-Scherwellen des Sensors, zum Beispiel als Verschiebung von Resonanzfrequenzen oder Phasen. Diese Änderung ergibt sich, wenn sich Liganden an die Rezeptoren heften, die auf dem Sensor immobilisiert werden (Schritt 5). Alternativ zu Love-Wave-Sensoren können auch Sensoren eingesetzt werden, die Änderungen anderer physikalischer Parameter detektieren (Fluoreszenz, elektrochemische Änderungen).

### 5. Immobilisierung der Rezeptoren:

Die Rezeptoren werden an sogenannte self-assembled monolayers (SAMs) gekoppelt. Typischerweise werden die SAMs wenn der Sensor (Schritt 4) mit Gold beschichtet ist aus substituierten Alkanthiolen oder wenn der Sensor eine Oberfläche aus Silizium-Dioxid hat aus Silanverbindungen hergestellt. Die SAM-Moleküle binden kovalent an die Oberfläche des Sensors aus Schritt 4. Die Aptamere werden ihrerseits terminal an die SAM-Moleküle gekoppelt, zum Beispiel unter Ausbildung von Bindungen der Typen Carbonsäure-Amid, Carbonsäure-Ester oder Phosphosäure-Amid. Diese Kopplungen sind reversibel, was die Wiederverwendung des Sensors erleichtert und beim Messvorgang genutzt werden kann (Schritt 6).

### 6. Messung der Gesamtmasse der Liganden:

Der Analyt, zum Beispiel ein Zell-Lysat, wird auf die Sensoren pipettiert. Durch Waschen werden jene Bestandteile des Analyten entfernt, die unspezifisch an die Rezeptoren binden. Die Masse der Liganden, die auf dem Sensor durch Rezeptoren gebunden wurden, werden über die Änderung im Frequenzspektrum der Oberflächenwellen des Love-Wave-Sensors oder durch Änderung anderer geeigneter Messgrößen bei Verwendung eines anderen Sensors gemessen. Es sind vor allem zwei Fehlerquellen bei der Messung der Gesamtmasse zu beachten:

Zum einen haften mitunter Teile der Probe unspezifisch am SAM und verfälschen dadurch die Messung; zum anderen kann das Messsignal bei sehr kleinen und leichten Liganden im Rauschen untergehen. Zur Erhöhung der Sensitivität im ersten Fall kann folgendes Verfahren angewendet werden: Im Schritt 5 werden die Rezeptoren über spaltbare Bindungen an die Unterlage gekoppelt. Nach dem ersten Waschen und Messen werden die Rezeptor-Ligand-Komplexe durch Auftrennen dieser Bindung abgespalten und die Sensor-Oberfläche zum zweiten Mal gewaschen. Der Überstand enthält dann die Rezeptor-Ligand-Komplexe, während auf dem Sensor die unspezifisch bindenden Teile des Analyten zurückbleiben. Die Masse der Rezeptor-Ligand Komplexe ergibt sich aus der Differenz der Massen vor und nach dem zweiten Waschen.

Ist die geringe Masse des Liganden die Hauptfehlerquelle, kann die Sensitivität folgendermaßen erhöht werden: Eine molekulare Signalverstärkung wird durch Verwendung spezieller Rezeptoren erreicht, die katalytisch aktiv sind und deren Aktivität durch den Liganden allosterisch reguliert wird. Die katalytische Aktivität kann zum Beispiel darin bestehen, dass nach Bindung eines Liganden der Rezeptor einen Teil seiner selbst abspaltet. Ist der Ligand leicht, das abgespaltene Stück aber schwer, so tritt eine Signalverstärkung ein: Die Masse nimmt nach Bindung des Liganden deutlich ab.

### 7. Messung der Molekülmassen und Bestimmung der Mengen der Liganden:

Je spezifischer die Rezeptoren sind, umso genauer ist das Ensemble der Liganden, die sich daran heften, definiert. Es werden also hochspezifische Rezeptoren zur Detektion von Liganden genutzt, die sich nur geringfügig unterscheiden, zum Beispiel in einer post-translationalen Modifikation. Rezeptoren mit geringerer Spezifität werden dagegen zur Entdeckung neuer Liganden verwendet. So zum Beispiel werden Rezeptoren verwendet, die nur ein kleines Epitop auf Protein-Liganden erkennen zur Entdeckung von Proteinen, die dieses Epitop aufweisen. In beiden Fällen werden die tatsächlich gebundenen Liganden in einem zweiten Analyse-Schritt mit Hilfe der Massenspektrometrie identifiziert. Dazu werden die molekularen Massen der Liganden oder die Massen von Ligand-Fragmenten gemessen. Die Fragmentierung wird mit verschiedenen chemischen und physikalischen Techniken erreicht. Im Falle von Proteinen wird sie durch gezielte chemische. Zerlegung erreich, zum Beispiel durch spezifische enzymatische Proteolyse, und danach durch Zerfall der Peptide im Massenspektrometer (Gatlin et al 2000 Anal Chem 72:757; Chaurand et al 1999 J Am Soc Mass Spec 10:91). Zur Vermeidung von Probenverlusten beim Transfer zum Massenspektrometer finden Proteolyse und Auslesen der Probe mit dem Massenspektrometer direkt auf dem Sensor statt, mit dem im Schritt 6 die Gesamtmasse gemessen wurde. Im einfachsten Falle der Belegung eines Sensors mit genau einer Liganden-Spezies wird die Menge der gebundenen Liganden folgendermaßen berechnet: Mit Schritt 6 wird die Gesamtmasse M aller auf dem Sensor gebundenen Ligandenmoleküle und aus Schritt 7 die Masse m des einzelnen Ligandenmoleküls bestimmt. Damit entspricht die Menge der gebundenen Ligandenmoleküle M = m. Liegen zwei Ligandensorten vor, so kann mit dem Love-Wave-Sensor die Gesamtmasse M aller Ligandenmoleküle beider Sorten bestimmt werden und mit der Massenspektrometrie (siehe zum Beispiel Cohen et al 2000 Anal Chem 72:574) sowohl die Massen m1 und m2 der einzelnen Ligandenmoleküle, als auch das Verhältnis R der Mengen der Liganden der zweiten und ersten Sorte bestimmt werden; daraus ergibt sich die Menge der ersten Sorte als M = (m1 + R _ m2). Analoges gilt für drei oder mehr Sorten. Das erfindungsgemässe Verfahren erlaubt also die direkte Quantifizierung der Mengen von Liganden beliebiger Sorten.

### 8. Vergleich mit Referenz:

Die Konzentrationen der gebundenen Moleküle und vorkommende Sorten werden verglichen mit entsprechenden Daten aus Referenzproben. Zum Beispiel werden Daten aus Lysat kranker Zellen mit solchen aus gesundem Gewebe verglichen. Der Vergleich der Messwerte aus Schritt 6 mit entsprechenden Werten von Referenzproben mit bekannter Konzentration des Liganden erlaubt die Bestimmung der Konzentration des Liganden in der biologischen Probe. Für manche Anwendungen, wie zum Beispiel das Finden von Zielproteinen für Wirkstoffe, werden im allgemeinen die Schritte 1 bis 8 mit neuen Liganden und Rezeptoren mehrfach wiederholt. Um eine hohe Effizienz des Verfahrens zu erreichen, ist es vorteilhaft, die Anzahl der Wiederholungen zu minimieren, besonders das Durchlaufen des vergleichsweise aufwändigen Schrittes 2. Dazu wird das vorhandene Wissen über metabolische und regulatorische Netzwerke und sonstige experimentelle Daten genutzt, die Hinweise geben auf Unterschiede der Häufigkeiten von Proteinen oder anderen Molekülen zwischen gesundem und krankem Gewebe. Ausgehend von diesen Daten, ist es möglich zum Beispiel durch mehrfaches Wiederholen der Schritte 1 bis 8 solche Liganden zu finden, bei denen die Differenz der Konzentrationen in gesundem und krankem Gewebe maximal ist. Diese Liganden sind geeignete Ziele für Therapien oder wechselwirken direkt mit solchen Ziel-Molekülen. Im Idealfall eignen sich die Rezeptoren, an die sich diese Liganden gebunden haben, als Wirkstoffe.

Anhand der Figuren wird nachfolgend ein Ausführungsbeispiel der Erfindung erklärt. Es zeigen
- **Figur 1**: einen Querschnitt durch einen Love-Wave-Sensor mit Aptameren als immobilisierte Rezeptoren,
- **Figur 2**: ein Schema eines Mikrowaage-Arrays im Anwendungsbeispiel. Dabei werden je fünf Aptamere gegen jedes der neun Virusproteine VP1, ...VP9 gezüchtet. Jeder Array hat somit 45 Aptamersensoren.
- **Figur 3**: eine Massen Messung mit der Mikrowaage: Je tiefer der Grauton, desto größer die Gesamtmasse auf dem Sensor. Die größte Massezunahme wird auf den Sensoren gemessen, auf denen Aptamer gegen VP4 immobilisiert wurde. Dort bindet VP4 im Komplex mit dem TF. VP4 ist somit das gesuchte Virusprotein. Die Aptamere A3 und A4 binden dagegen VP4 kompetitiv zu TF. Diese Aptamere sind damit Kandidaten für antivirale Wirkstoffe.

Das folgende Beispiel zeigt, wie eine Ausprägung des oben beschriebenen Verfahrens eine neuartige Anwendung möglich macht. Ziel ist in diesem Beispiel die Entwicklung eines Wirkstoffs gegen ein human-pathogenes Virus. Unter den Virus-Proteinen (VPs) ist eines (VPX), das mit einem humanen Transkriptionsfaktor (TF) wechselwirkt und dadurch die Produktion von Virus-Partikeln verstärken. Zwar sind alle Proteine des Virus bekannt, aber es ist nicht bekannt, welches der VPs mit welchem TF wechselwirkt. Das erste Teilproblem bei der Suche nach einem Wirkstoff ist daher die Identifikation von VPX und dem damit wechselwirkenden TF. Mit dem erfindungsgemässen Verfahren wird dieses Problem zum Beispiel wie folgt gelöst:

Im ersten Schritt werden Aptamere (Rezeptoren) gegen alle VPs (Liganden) gezüchtet, und zwar züchtet man mehrere Aptamere gegen jedes VP.

Im nächsten Schritt werden die Aptamere auf einer Anordnung von Sensoren immobilisiert, und zwar mit je einer Aptamer-Sorte pro Sensor (Abb. 2). Die Sensoren sind geschichtete Love-Wave-Sensoren (Abb. 1): Auf ein SiO2 -Substrat ist eine Chromschicht aufgebracht, auf dieser wiederum eine Goldschicht; ein self-assembled Monolayer aus substituierten Alkanthiolen ist kovalent an die Goldschicht gebunden; schliesslich werden DNA-Aptamere über eine Carbonsäure-AmidBindung an die Alkanthiole gekoppelt.

Im nächsten Schritt wird Lysat Virus-infizierter Zellen über jeden Sensor der Anordnung pipettiert und es werden schwach und unspezifisch bindende Moleküle durch Waschen entfernt. Zum Beispiel lösen sich bei Erhöhung der Konzentration eines Detergens oder bei der Erhöhung der Salzkonzentration zuerst schwach bindende Moleküle. Dabei dient das Signal des Love-Wave-Sensors zur Kontrolle des Wascherfolges: Sind die schwach gebundenen Moleküle von der Oberfläche des Sensors entfernt, ändert sich das Mess-Signal nur noch langsam mit steigender Konzentration an Detergens oder Salz.

Im nächsten Schritt binden Aptamere auf einem Sensor oder auf mehreren Sensoren der Anordnung VPs, teilweise im Komplex mit TFs, was über die Frequenzänderung von Scherwellen auf der Oberfläche dieser Love-Wave-Sensoren gemessen wird. Zur Normierung der Messwerte wird jeweils VPs in bekannter Konzentration hinzu gegeben, sodass alle Aptamere mit VPs abgesättigt sind. Danach wird erneut die Gesamtmasse der Liganden auf jedem Sensor gemessen. Die Massendifferenz zur vorherigen Messung ist proportional zur Anzahl von Rezeptoren, die nicht durch Liganden aus dem Lysat besetzt wurden. Je kleiner diese Zahl, umso höher ist die Ligand-Konzentration im Lysat.

Auf einigen Sensoren ist nach dem Absättigen der Aptamere mit VPs im Schritt 4 die Gesamtmasse größer als die Masse der VPs alleine. Unter den Liganden auf diesen Sensoren ist wahrscheinlich das gesuchte VPX im Komplex mit TFs (Abb. 3). Die tatsächliche Identität der Liganden wird massenspektrometrisch bestimmt. Dazu werden die Liganden auf diesen Sensoren durch spezifische Proteolyse, zum Beispiel mit Trypsin, verdaut. Die Massen der Peptid-Fragmente werden dann im Massenspektrometer gemessen und die Proteine, insbesondere die TFs, durch Suche in Datenbanken ("Peptide-Mass-Fingerprint") identifiziert. Damit ist VPX und die mit ihm wechselwirkenden TFs bekannt und das erste Teilproblem gelöst.

Im Schritt 1 wurden mehrere Aptamere gegen VPX gezüchtet, die sich zum Beispiel in ihrer Bindestelle auf VPX unterscheiden. Aptamere, die mit dem TF um die gleiche Bindestelle an VPX konkurrieren, können idealerweise als Wirkstoffe gegen das Virus verwendet werden. In den Schritten 4 und 5 zeichnen sich solche Aptamere dadurch aus, dass die Sensoren, auf denen diese Aptamere immobilisiert wurden, zwar VPX binden, aber keine Komplexe von VPX und TFs (Abb. 3).

### Literatur

Harding G.L., Du J., Dencher P.R., Barnett D., Howe E. (1997): Love wave acoustic immunosensor operating in liquid, Sensors and Actuators A 61, 279-286.

## Patentansprüche

1. Verfahren zum Nachweis eines in einer biologischen Probe befindlichen und in molekularer Form vorliegenden Analyten, wobei ein Sensor mit einem den Analyten bindenden Substrat versehen und in einem ersten Verfahrensschritt der Sensor mit dem Analyten beaufschlagt wird und wobei zum quantitativen Nachweis in einem nachfolgenden Verfahrensschritt die vom Sensor gebundene Gesamtmasse des Analyten gemessen wird,
**dadurch gekennzeichnet, daß** zum qualitativen Nachweis in einem weiteren Schritt das molekulare Gewicht des die Gesamtmasse bildenden Analyten direkt auf dem Sensor mittels eines Massenspektrometers gemessen wird.

2. Verfahren nach Anspruch 1,
**dadurch gekennzeichnet, daß** die Gesamtmasse mittels einer Mikrowaage, insbesondere mittels eines Love-Wave-Sensors, gemessen wird.

3. Verfahren nach Anspruch 1 oder 2,
**dadurch gekennzeichnet, daß** mehrere Sensoren in einem gemeinsamen Sensorarray angeordnet werden.

4. Verfahren nach Anspruch 1,
**dadurch gekennzeichnet, daß** die gesamte im Sensor gebundene Masse mit einem Massenspektrometer untersucht wird.

5. Verfahren nach einem der vorherigen Ansprüche,
**dadurch gekennzeichnet, daß** mehrere Analyten synchron gemessen werden.

6. Verfahren nach einem der vorherigen Ansprüche,
**dadurch gekennzeichnet, daß** im Sensorarray verschiedene Substrate verwendet werden, die den gleichen Analyten auf verschiedene Weise binden.

7. Verfahren nach einem der vorherigen Ansprüche,
**dadurch gekennzeichnet, daß** im Sensorarray verschiedene Substrate verwendet werden, die verschiedene Analyte binden.

8. Verfahren nach einem der vorherigen Ansprüche,
**dadurch gekennzeichnet, daß** der Analyt Teil eines molekularen Komplexes aus mehreren Molekülen ist.

9. Verfahren nach einem der vorherigen Ansprüche,
**dadurch gekennzeichnet, daß** das Substrat reversibel auf dem Sensor immobilisiert ist.

10. Verfahren nach einem der vorherigen Ansprüche,
**dadurch gekennzeichnet, daß** die Messung unter physiologischen Bedingungen durchgeführt wird .

11. Verfahren nach einem der vorherigen Ansprüche,
**dadurch gekennzeichnet, daß** das Substrat so ausgewählt wird, dass es den Analyten an einem Epitop bindet.

12. Verfahren nach einem der vorherigen Ansprüche,
**dadurch gekennzeichnet, daß** die Mengenverhältnisse unterschiedlicher Sorten von Analytmolekülen über die Signalintensitäten im Massenspektrum bestimmt werden.

13. Verfahren nach einem der vorherigen Ansprüche,
**dadurch gekennzeichnet, daß** die absoluten Mengen unterschiedlicher Analytmoleküle aus der Gesamtmasse des Analyten auf dem Sensor, den Molekülmassen und den Mengenverhältnissen bestimmt werden.

14. Verfahren nach einem der vorherigen Ansprüche,
**dadurch gekennzeichnet, daß** die Belegung des Substrates mit Analytmolekülen bestimmt wird durch Differenzmessung zwischen eigentlichem Analyten und zusätzlicher Absättigung des Substrates mit Kalibrierungsprobe bekannter Analytkonzentration.

15. Verfahren nach einem der vorherigen Ansprüche,
**dadurch gekennzeichnet, daß** ein Substrat aus Oligonukleotiden oder Proteinen gewählt wird.

16. Verfahren nach einem der vorherigen Ansprüche,
**dadurch gekennzeichnet, daß** das Messsignal der Gesamtmasse beim Waschen des Sensors zur Optimierung der Probenvorbereitung für die Massenspektrometrie genutzt wird.

17. Verfahren nach einem der vorherigen Ansprüche,
**dadurch gekennzeichnet, daß** vergleichende Messungen an biologischen Proben unter verschiedenen Bedingungen oder unterschiedlicher Herkunft durchgeführt werden.

18. Verfahren nach einem der vorherigen Ansprüche,
**dadurch gekennzeichnet, daß** Messsignale zusammen mit bekannten biomolekularen Wechselwirkungen und mit vergleichenden Messungen dazu verwendet werden, neue Substrate auszuwählen, die an andere Analyte binden, und dann neue Messungen mit diesen Substraten durchgeführt werden.

## Claims

1. A method for detecting an analyte which is present in a molecular form in a biological sample, such that a sensor is provided with a substrate that binds the analyte, and the sensor is exposed to the analyte in a first process step, and for quantitative detection, the total mass of the analyte bound by the sensor is measured in a subsequent process step,
**characterized in that**
for qualitative detection in another step, the molecular weight of the analyte forming the total mass is measured directly on the sensor by means of a mass spectrometer.

2. The method according to Claim 1,
**characterized in that**
the total mass is measured by a microbalance, in particular by a Love wave sensor.

3. The method according to Claim 1 or 2,
**characterized in that**
several sensors are arranged in a shared sensor array.

4. The method according to Claim 1,
**characterized in that**
the total mass bound in the sensor is analyzed in a mass spectrometer.

5. The method according to any one of the preceding claims,
**characterized in that**
several analytes are measured in synchronization.

6. The method according to any one of the preceding claims,
**characterized in that**
different substrates which bond the same analyte in different ways are used in the sensor array.

7. The method according to any one of the preceding claims,
**characterized in that**
different substrates which bind different analytes are used in the sensor array.

8. The method according to any one of the preceding claims,
**characterized in that**
the analyte is part of a molecular complex of several molecules.

9. The method according to any one of the preceding claims,
**characterized in that**
the substrate is reversibly immobilized on the sensor.

10. The method according to any one of the preceding claims,
**characterized in that**
the measurement is performed under physiological conditions.

11. The method according to any one of the preceding claims,
**characterized in that**
the substrate is selected so that it binds the analyte to an epitope.

12. The method according to any one of the preceding claims,
**characterized in that**
the quantity ratios of different types of analyte molecules are determined based on the signal intensities in the mass spectrum.

13. The method according to any one of the preceding claims,
**characterized in that**
the absolute quantities of different analyte molecules are determined from the total mass of the analyte on the sensor, the molecular masses and the quantity ratios.

14. The method according to any one of the preceding claims,
**characterized in that**
the occupancy of the substrate with analyte molecules is determined by differential measurement between the actual analyte and the additional saturation of the substrate with calibration sample of a known analyte concentration.

15. The method according to any one of the preceding claims,
**characterized in that**
a substrate of oligonucleotides or proteins is selected.

16. The method according to any one of the preceding claims,
**characterized in that**
the measurement signal of the total mass is utilized to optimize the sample preparation for mass spectrometry in washing the sensor.

17. The method according to any one of the preceding claims,
**characterized in that**
comparative measurements are performed on biological samples of various origins or under various conditions.

18. The method according to any one of the preceding claims,
**characterized in that**
measurement signals together with known biomolecular interactions and comparative measurements are used to select new substrates that bind to other analytes and then new measurements are performed with these substrates.

## Revendications

1. Procédé pour la détection d'un analyte contenu dans un échantillon biologique et se présentant sous une forme moléculaire, consistant à pourvoir un capteur d'un substrat qui retient ledit analyte et à charger, dans une première étape du procédé, le capteur avec l'analyte et à mesurer, dans une étape suivante du procédé, la masse totale de l'analyte retenue par le capteur pour ainsi effectuer une détection quantitative.
**caractérisé en ce que**, dans une autre étape, la détection qualitative est réalisée en mesurant, directement sur le capteur, la masse moléculaire de l'analyte formant ladite masse totale au moyen d'un spectromètre de masse.

2. Procédé selon la revendication 1,
**caractérisé en ce que** la masse totale est mesurée au moyen d'une microbalance, notamment au moyen d'un capteur à ondes de Love.

3. Procédé selon les revendications 1 ou 2,
**caractérisé en ce que** plusieurs capteurs sont disposés dans une barrette de capteurs commune.

4. Procédé selon la revendication 1,
**caractérisé en ce que** l'intégralité de la masse retenue dans le capteur est analysée par un spectromètre de masse.

5. Procédé selon l'une des revendications précédentes,
**caractérisé en ce que** plusieurs analytes sont mesurés d'une manière synchrone.

6. Procédé selon l'une des revendications précédentes,
**caractérisé en ce que** différents substrats qui retiennent le même analyte de manières différentes sont utilisés dans la barrette de capteurs.

7. Procédé selon l'une des revendications précédentes,
**caractérisé en ce que** différents substrats qui retiennent différents analytes sont utilisés dans la barrette de capteurs.

8. Procédé selon l'une des revendications précédentes,
**caractérisé en ce que** l'analyte fait partie d'un complexe moléculaire constitué de plusieurs molécules.

9. Procédé selon l'une des revendications précédentes,
**caractérisé en ce que** le substrat est immobilisé sur le capteur d'une manière réversible.

10. Procédé selon l'une des revendications précédentes,
**caractérisé en ce que** la mesure est réalisée dans des conditions physiologiques.

11. Procédé selon l'une des revendications précédentes,
**caractérisé en ce que** le substrat est choisi tel qu'il retient l'analyte par un épitope.

12. Procédé selon l'une des revendications précédentes,
**caractérisé en ce que** les rapports quantitatifs entre différents types de molécules à analyser sont déterminés à travers les intensités des signaux relevés dans le spectre de masse.

13. Procédé selon l'une des revendications précédentes,
**caractérisé en ce que** les quantités absolues des différentes molécules à analyser sont déterminées à partir de la masse totale de l'analyte sur le capteur, des masses moléculaires et des rapports quantitatifs.

14. Procédé selon l'une des revendications précédentes,
**caractérisé en ce que** la charge du substrat en molécules à analyser est déterminée en mesurant la différence entre l'analyte tel quel et après une saturation supplémentaire du substrat avec un échantillon de calibration présentant une concentration connue de l'analyte.

15. Procédé selon l'une des revendications précédentes,
**caractérisé en ce que** l'on choisit un substrat constitué d'oligonucléotides ou de protéines.

16. Procédé selon l'une des revendications précédentes,
**caractérisé en ce que** le signal de la masse totale, qui est mesuré lors de la purge du capteur, est utilisé pour optimiser la préparation des échantillons pour la spectrométrie de masse.

17. Procédé selon l'une des revendications précédentes,
**caractérisé en ce que** des mesures comparatives sont réalisées sur des échantillons biologiques, les conditions ou les origines étant différentes.

18. Procédé selon l'une des revendications précédentes,
**caractérisé en ce que** les signaux mesurés sont utilisés, ensemble avec des interactions biomoléculaires connues et avec des mesures comparatives, pour sélectionner de nouveaux substrats entrant en liaison avec d'autres analytes et pour réaliser ensuite de nouvelles mesures aves ces substrats.
